# EUROPEAN PATENT SPECIFICATION

(11) **EP 2 514 438 B1**
(45) Date of publication and mention of the grant of the patent: **22.03.2017**
(21) Application number: 10837732.6
(22) Date of filing: 17.12.2010
(51) Int. Cl.: A61K 39/39, A61P 37/08

(54) **ADJUVANT CONTAINING ß-HEMATIN**
ß-HEMATIN ENTHALTENDES ADJUVANS
ADJUVANT CONTENANT DE LA ß-HÉMATINE

(30) Priority: 18.12.2009 JP 2009287709
(43) Date of publication of application: 24.10.2012
(73) Proprietor: Osaka University, Suita-shi Osaka 565-0871 (JP); Nippon Zenyaku Kogyo Co., Ltd., Koriyama-shi, Fukushima 963-0196 (JP)
(72) Inventor: AKIRA, Shizuo, Suita-shi Osaka 565-0871 (JP); ISHII, Ken, Suita-shi Osaka 565-0871 (JP); COBAN, Cevayir, Suita-shi Osaka 565-0871 (JP); TSUKUI, Toshihiro, Koriyama-shi Fukushima 963-0196 (JP); IGARI, Yoshikatsu, Koriyama-shi Fukushima 963-0196 (JP); OHATA, Keiichi, Koriyama-shi Fukushima 963-0196 (JP)
(74) Representative: Keirstead, Tanis Evelyne
(86) International application number: PCT/JP2010/073474
(87) International publication number: WO 2011/074711

(56) References cited:
- WO-A1-2006/061965
- WO-A1-2007/147255
- WO-A1-2009/057763
- BOHLE D SCOTT ET AL: "Phase homogeneity and crystal morphology of the malaria pigment beta-hematin", ACTA CRYSTALLOGRAPHICA SECTION D BIOLOGICAL CRYSTALLOGRAPHY, vol. 58, no. 10 Part 1, October 2002 (2002-10), pages 1752-1756, XP002696093, ISSN: 0907-4449
- COBAN CEVAYIR ET AL: "Toll-like receptor 9 mediates innate immune activation by the malaria pigment hemozoin", JOURNAL OF EXPERIMENTAL MEDICINE, vol. 201, no. 1, 3 January 2005 (2005-01-03), pages 19-25, XP002696094, ISSN: 0022-1007
- EGAN ET AL: "Recent advances in understanding the mechanism of hemozoin (malaria pigment) formation", JOURNAL OF INORGANIC BIOCHEMISTRY, ELSEVIER INC, US, vol. 102, no. 5-6, 1 May 2008 (2008-05-01) , pages 1288-1299, XP022612005, ISSN: 0162-0134, DOI: 10.1016/J.JINORGBIO.2007.12.004 [retrieved on 2007-12-23]
- EGAN T J ET AL: "Quinoline anti-malarial drugs inhibit spontaneous formation of beta-haematin (malaria pigment)", FEBS LETTERS, ELSEVIER, AMSTERDAM, NL, vol. 352, no. 1, 19 September 1994 (1994-09-19), pages 54-57, XP025576538, ISSN: 0014-5793, DOI: 10.1016/0014-5793(94)00921-X [retrieved on 1994-09-19]
- COBAN CEVAYIR ET AL: "Immunogenicity of Whole-Parasite Vaccines against Plasmodium falciparum Involves Malarial Hemozoin and Host TLR9", CELL HOST & MICROBE, vol. 7, no. 1, January 2010 (2010-01), pages 50-61, XP002696095, ISSN: 1931-3128
- COBAN CEVAYIR ET AL: "The Malarial Metabolite Hemozoin and Its Potential Use as a Vaccine Adjuvant", ALLERGOLOGY INTERNATIONAL, JAPANESE SOCIETY OF ALLERGOLOGY, JP, vol. 59, no. 2, 1 June 2010 (2010-06-01), pages 115-124, XP002615320, ISSN: 1323-8930, DOI: 10.2332/ALLERGOLINT.10-RAI-0194
- EGAN TIMOTHY J ET AL: "The mechanism of beta-hematin formation in acetate solution. Parallels between hemozoin formation and biomineralization processes", BIOCHEMISTRY, vol. 40, no. 1, 9 January 2000 (2000-01-09), pages 204-213, XP002696096, ISSN: 0006-2960
- SLATER A F G ET AL: "AN IRON CARBOXYLATE BOND LINKS THE HEME UNITS OF MALARIA PIGMENT", PROCEEDINGS OF THE NATIONAL ACADEMY OF SCIENCES OF THE UNITED STATES OF AMERICA, vol. 88, no. 2, 1991, pages 325-329, XP002696097, ISSN: 0027-8424
- JARAMILLO M. ET AL.: 'Hemozoin-inducible proinflammatory events in vivo: potential role in malaria infection.' JOURNAL OF IMMUNOLOGY vol. 172, no. 5, 2004, ISSN 0022-1767 pages 3101 - 3110
- JARAMILLO M. ET AL.: 'Hemozoin increases IFN-gamma-inducible macrophage nitric oxide generation through extracellular signal- regulated kinase- and NF-kappa B-dependent pathways.' JOURNAL OF IMMUNOLOGY vol. 171, no. 8, 2003, ISSN 0022-1767 pages 4243 - 4253
- JARAMILLO M. ET AL.: 'Hemozoin induces macrophage chemokine expression through oxidative stress-dependent and -independent mechanisms.' JOURNAL OF IMMUNOLOGY vol. 174, no. 1, 2005, ISSN 0022-1767 pages 475 - 484
- JARAMILLO M. ET AL.: 'Synthetic Plasmodium-like hemozoin activates the immune response: a morphology - function study.' PLOS ONE vol. 4, no. 9, September 2009, ISSN 1932-6203

## Description

### Technical Field

The present invention relates to a method for preparing a vaccine adjuvant composition.

### Background Art

A potent vaccine contains not only a protective antigen, which induces long time adaptive immunity but also an adjuvant component, which activates the natural immune system.

To protect against and interfere with malaria infection, lately, a whole parasite vaccine strategy targeting the erythrocytic-stage has attracted attention. However, an adjuvant component important for immunogenicity and its functional mechanism have not yet been known.

Hemozoin is a hydrophobic heme dimer aggregate (crystal), which is a detoxication product of a heme molecule present in the food vacuole in *Plasmodium* protozoan and formed through digestion of host hemoglobin by *Plasmodium* protozoan. Similar to CpG DNA, hemozoin has been reported to serve as a ligand for Toll-like receptor 9 (TLR9). Hemozoin synthesized from hemin chloride is called β-hematin (see Non Patent Literature 1).

It is reported that hemozoin activates spleen cells and dendritic cells of mice *in vitro* (see Patent Literature 1). It is also reported that hemozoin has an adjuvant effect on antibody production of ribonuclease A in mice (see Patent Literature 2).

In addition, it is reported that β-hematin has an effect as an adjuvant on DNA vaccines (see Non Patent Literature 2) and also reported that β-hematin functions as a ligand for molecules other than a TLR9 DNA molecule (non-methylated DNA chain called as a CpG motif) (see Non Patent Literature 3).

It has been further reported that hemozoin is used as a vaccine adjuvant *in vivo* for potentiating the effect of allergen vaccines or vaccines against bacterial and viral infections (see Patent Literature 3).

Methods for producing synthetic hemozoin crystals for preparing e.g. a vaccine adjuvant composition are described in WO2007/147255 and Jaramillo et al., 2009, PLOS ONE, 4(9).

### Citation List

### Patent Literatures

Patent Literature 1: International Publication No. WO2006/061965
Patent Literature 2: U. S. Pat. No. 5849307
Patent Literature 3: International Publication No. WO2009/057763

### Non Patent Literatures

Non Patent Literature 1: Slater et al., Proc. Natl. Acad, Sci. U.S.A. 88: 325-329, 1991
Non Patent Literature 2: Infect Immun. 2002 Jul; 70 (7): 3939-43
Non Patent Literature 3: J Exp Med. 2005 Jan 3; 201 (1): 19-25

### Summary of Invention

An object of the present invention is to provide a method for preparing a vaccine adjuvant composition containing β-hematin.

The present inventors found that synthetic hemozoin, which is a heme crystal produced during the infection period of host erythrocytes with *Plasmodium falciparum* (Pf), separately activates Toll-like receptor (TLR) 9 and inflammasome, which is an apoptosis-associated speck-like protein containing a CARD (ASC) without requiring the presence of DNA, thereby exhibiting potent adjuvant activity.

Furthermore, the present inventors prepared samples by dissolving hemin chloride in a sodium hydroxide solution; adding a small amount of hydrochloric acid to the resultant solution, adding acetic acid to the resultant solution at 60°C until pH reached about pH 4.8, keeping the resultant mixture still under room temperature for 16 hours for reaction to occur, centrifuging the reaction mixture to obtain a precipitate, washing the precipitate with a weakly basic bicarbonic acid solution (pH about 9) containing 2% of sodium dodecyl sulfate (SDS), replacing the solution with water, and fractioning the precipitate by centrifugation, and measured the samples by a wet laser diffraction light scattering particle size distribution measuring apparatus. Using the sample thus measured, the adjuvant effect of each size was checked. As a result, they found that a fraction of particles having a size of 50 to 200 nm has the most potent adjuvant effect and accomplished the present invention.

More specifically, the present invention is as follows.
[1] A method for preparing a vaccine adjuvant composition containing a β-hematin crystal, including dissolving hemin chloride in an aqueous NaOH solution, adding a small amount of hydrochloric acid to the resultant solution, adding dropwise acetic acid to the resultant solution at room temperature to 60°C to adjust pH to 4 to 6, keeping the solution mixture still at room temperature to 40°C for 1 to 24 hours for reaction to occur, centrifuging the resultant mixture to obtain a β-hematin crystal, washing the β-hematin crystal with an SDS-containing weakly basic solution of about pH9, and pulverizing the washed -hematin crystal by autoclaving to obtain a beta-hematin crystal having an average particle size of 50 to 500 nm.
[2] The method for preparing a vaccine adjuvant composition comprising a -hematin crystal according to item [1], wherein the washed -hematin crystal is pulverized by autoclaving to obtain a -hematin crystal having an average particle size of 50 to 300 nm.
[3] The method for preparing a vaccine adjuvant composition comprising a -hematin crystal according to item [1], wherein the washed -hematin crystal is pulverized by autoclaving to obtain a -hematin crystal having an average particle size of 50 to 200 nm.

By using the vaccine adjuvant composition containing β-hematin disclosed herein in combination with an allergen vaccine or a vaccine against an infection with a pathogen such as a bacterium, a virus, a rickettsia or a parasite, the antibody titer against the pathogen increases *in vivo* compared to the case where no adjuvant is used in combination, with the result that an allergy disease and an infection can be effectively prevented or treated.

### Brief Description of the Drawings

Figure 1 is a graph showing adjuvant effect of a Pf crude extract depending upon TLR9, more specifically, showing an anti-Pf extraction specific IgG antibody production in the serum.
Figure 2 is a graph showing adjuvant effect of a Pf crude extract depending upon TLR9, more specifically, showing an anti-Pf extraction specific IgG2c antibody production in the serum.
Figure 3 is a graph showing adjuvant effect of a Pf crude extract depending upon TLR9, more specifically, showing IFNγ production of cultured spleen cells.
Figure 4 shows FESEM images of synthetic hemozoin synthesized by two methods (Method 1 and Method 2 in Example); Figure 4A shows hemozoin synthesized in accordance with Method 1, and Figure 4B shows hemozoin synthesized in accordance with Method 2. The scale bar is 500 nm.
Figure 5 is a graph showing an OVA specific IgG response in the sera of C57B/6 mice immunized with an OVA antigen by subcutaneous injection in the presence or absence of synthetic hemozoin synthesized by Method 1 or Method 2 (measured by ELISA).
Figure 6 is a graph showing a particle size distribution (rate of the numbers of particles) obtained in fractionating the synthetic hemozoin synthesized by Method 1.
Figure 7 is a graph showing the adjuvant effect of synthetic hemozoin different in size (50 to 200 nm and 2 to 20 µm) or hemin (50 nm or less), more specifically, an OVA specific IgG antibody response in the sera of C57B/6 mice immunized with OVA.
Figure 8 is a graph showing potent adjuvant activity and concentration dependency of synthetic hemozoin. Wild-type mice were immunized with a model antigen (10 µg), HSA, together with synthetic hemozoin different in concentration and boosted with the same amount of HSA and synthetic hemozoin, 10 days later. HSA specific IgG response in the sera a week after the booster immunization (measured by ELISA) is shown.
Figure 9 is a graph showing potent adjuvant activity of synthetic hemozoin. Wild-type mice were immunized with a model antigen (10 µg), HSA, together with synthetic hemozoin different in concentration and boosted with the same amount of HSA and synthetic hemozoin, 10 days later. An isotype of HSA specific IgG in the sera a week after the booster immunization is shown.
Figure 10 is a graph showing the adjuvant effects of alum, CpG DNA and synthetic hemozoin for comparison. Mice were immunized with OVA antigen, alum (200 µg), CpG DNA (50 µg) and synthetic hemozoin (800 µg) and boosted 10 days later. The OVA specific IgG response in the sera was measured by ELISA.
Figure 11 is a graph showing the adjuvant effect of synthetic hemozoin on a whole-blood malaria antigen. Wild-type mice were immunized with 10 µg of a Pf crude extract and 800 µg of synthetic hemozoin and boosted with the same amount of Pf crude extract and synthetic hemozoin. The Pf crude extract specific IgG response in the sera was measured by ELISA a week after the booster immunization.
Figure 12 is a graph showing the effect of synthetic hemozoin on mite allergy of a dog; more specifically showing anti-Derf2 specific IgG2 and IgG1 responses in the sera of beagle dogs immunized with Derf2 alone, a mixture preparation of Derf2 and alum (500 µg) and a mixture preparation of Derf2, alum and synthetic hemozoin.
Figure 13 is a graph showing the effect of synthetic hemozoin on allergy of a dog, more specifically, showing anti-Derf2 specific IgE response in the sera of beagle dogs after Derf2 sensitization.

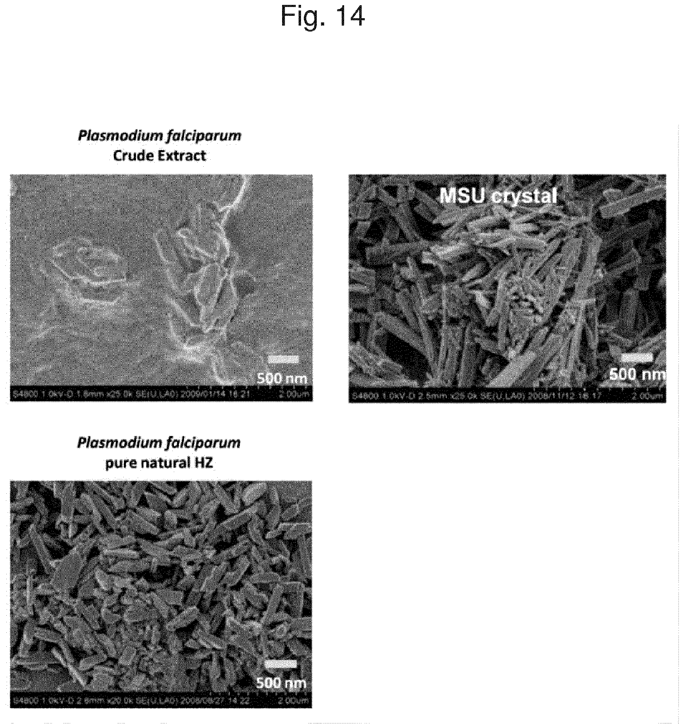

Figure 14 shows FESEM images of a Pf crude extract containing a protein, a lipid and a membrane, pure natural hemozoin extracted from Pf and an MSU crystal. Scale bar is 500 nm.
Figure 15 is a graph showing the adjuvant effect of hemozoin. Balb/c mice were immunized with OVA alone or OVA in combination with synthetic hemozoin by subcutaneous injection and boosted in the same amount, 10 days later. The results are shown.
Figure 16 is a graph showing the adjuvant effect of hemozoin. Balb/c mice were immunized with OVA alone or OVA in combination with synthetic hemozoin by nasal administration and boosted in the same amount, 10 days later. The results are shown.
Figure 17 shows purity of a synthetic hemozoin preparation, more specifically, TLC (thin layer chromatography) results showing contamination of the synthetic hemozoin preparation with hemin. Synthetic hemozoin and hemin, which corresponds to 10% of synthetic hemozoin in terms of hem iron equivalent amount, were spotted on a silica gel plate and developed with methanol. On the right side of the TLC plate, a thin band of a hemin contaminant in synthetic hemozoin is observed.
Figure 18 is a graph showing the results of FT-IR of hemin and synthetic hemozoin. The peak of hemin shifts, which suggests that β-hematin was produced.
Figure 19 shows the diffraction pattern of X-ray analysis of synthetic hemozoin powder.
Figure 20 is a graph showing the adjuvant effects between 2 lots of β-hematin samples having a large average particle size (2 to 50 µm) and a small average particle size (20 to 500 nm), more specifically, showing production of anti-OVA (egg albumin) specific IgG antibody in C57BL6 mouse sera at the time of 4 mM subcutaneous administration.
Figure 21 is a graph showing the adjuvant effects between 2 lots of β-hematin samples having a large average particle size (2 to 50 µm) and a small average particle size (20 to 500 nm), more specifically, showing production of anti-OVA (egg albumin) specific IgG2a antibody in C57BL6 mouse sera at the time of 4 mM subcutaneous administration.
Figure 22 is a graph showing the adjuvant effects between 2 lots of β-hematin samples having a large average particle size (2 to 50 µm) and a small average particle size (20 to 500 nm), more specifically, showing production of anti-OVA (egg albumin) specific IgG2b antibody in C57BL6 mouse sera at the time of 4 mM subcutaneous administration.
Figure 23 is a graph showing the adjuvant effects of β-hematin samples having a large average particle size (2 to 50 µm) and a small average particle size (20 to 500 nm), more specifically, showing production of anti-HSA (human serum albumin) specific IgG antibody in Balb/c mouse sera at the time of 4 mM subcutaneous administration.
Figure 24 is a graph showing the adjuvant effects of β-hematin samples having a large average particle size (2 to 50 µm) and a small average particle size (20 to 500 nm), more specifically, showing production of anti-OVA specific IgG antibody in C57BL6 mouse sera at the time of 4 mM subcutaneous administration.
Figure 25 is a graph showing the adjuvant effects of β-hematin samples having a large average particle size (2 to 50 µm) and a small average particle size (20 to 500 nm), more specifically, showing production of anti-OVA specific IgG antibody in C57BL6 mouse sera at the time of 4 mM intraperitoneal administration.
Figure 26 is a graph showing the adjuvant effects of β-hematin samples having a large average particle size (Big, 2 to 50 µm) and a small average particle size (Small, 20 to 500 nm) synthesized each from raw materials manufactured by Fluka, more specifically, showing production of anti-HSA specific IgG antibody in Balb/c mouse sera at the time of 4 mM subcutaneous administration.
Figure 27 is a graph showing the adjuvant effects of β-hematin samples having a large average particle size (Big, 2 to 50 µm) and a small average particle size (Small, 20 to 500 nm) synthesized from raw materials of TCI (Tokyo Chemical Industry Co., Ltd.), more specifically, showing production of anti-HSA specific IgG antibody in Balb/c mouse sera at the time of 4 mM subcutaneous administration.
Figure 28 is a graph showing total IgG level in blood when a β-hematin sample autoclaved at 121°C for 20 minutes was administrated to mice.
Figure 29 is a graph showing IgG2a, IgG2b, IgG2c and IgG3 in blood when a β-hematin sample autoclaved at 121°C for 20 minutes was administrated to mice.
Figure 30 is a graph showing the particle size distribution of a β-hematin sample autoclaved at 121°C for 20 minutes.
Figure 31 shows micrographic images of a β-hematin sample taken by a stereoscopic microscope before and after an autoclave treatment at 121°C for 20 minutes.

### Description of Embodiments

The present invention will be described in detail below.

β-hematin, which is a component constituting a vaccine adjuvant composition , is synthetic hemozoin, which can be synthesized from hemin chloride in accordance with the following method.

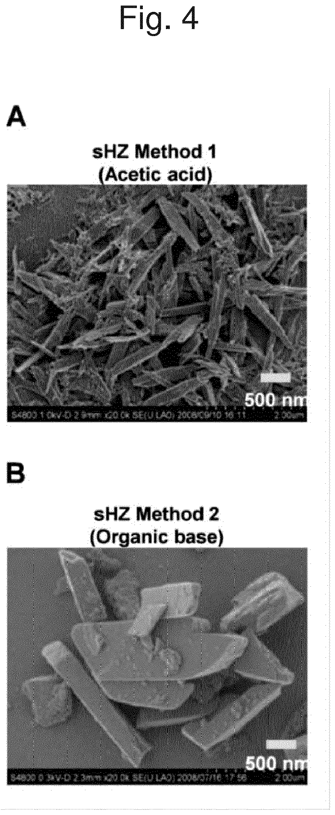

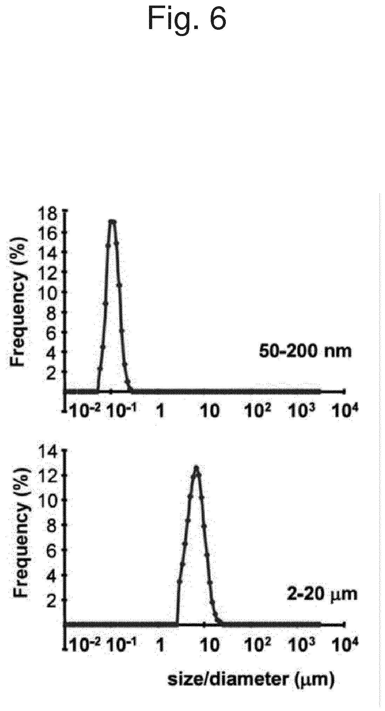

Hemin chloride (45 mg) is dissolved in a 1N aqueous NaOH solution (4.5 mL), to which, a 1N aqueous HCl solution (0.45 mL) is added. To the resultant solution, acetic acid is added dropwise at room temperature to 60°C to adjust pH to 4 to 6, preferably 4.5 to 5 and further preferably 4.8. The solution mixture is kept still at room temperature to 40°C for 1 to 24 hours for reaction to occur, and centrifuged to obtain a precipitate. The precipitate was washed with a 2% SDS containing weakly basic solution (about pH9), for example, 0.1 M sodium bicarbonate buffer (pH9.1) and substituted with pure water. In this manner, a β-hematin crystal can be obtained. The β-hematin thus prepared is fractionated based on particle size and β-hematin having a small particle size is used. An average particle size can be determined by, for example, a wet laser diffraction light scattering particle size distribution measuring apparatus. The size of a β-hematin crystal that the vaccine composition contains is 20 to 1000 nm, preferably 20 to 500 nm and further preferably 50 to 200 nm in terms of an average particle size. Note that the β-hematin thus prepared has a crystal structure shown in Figure 4A. The β-hematin is a dispersed single crystalline (primary particle) having a size of nm order, which is obtained by fractionating β-hematin formed of aggregates into single crystals in the order of nm and determining an average particle size thereof (Figure 6). The average particle size of the aggregate is 2 to 50 µm and an average particle size of the primary particle obtained by dispersing it, is 20 to 500 nm and preferably 50 to 200 nm.

The β-hematin synthesized is dissolved in a 20 mM sodium hydroxide containing 2% SDS and kept still for 2 hours at room temperature for reaction to occur and then the absorbance at 400 nm is measured. In this manner, quantitative determination can be made. The quantitative determination can be made in accordance with a method, for example, described in Proc. Natl. Acad. Sci. U.S.A. 93: 11865-11870, 1996.

Furthermore, hemin chloride is treated with HCl and acetic acid as described above and the resultant precipitate may be autoclaved. The autoclave treatment may be performed by an autoclave usually used for sterilization treatment. The autoclave treatment may be performed, for example, at a temperature of 100°C or more for 1 to 99 minutes, preferably at 105 to 135°C for 1 to 99 minutes, and further preferably at 121°C for 10 to 30 minutes. The autoclave treatment is usually performed under 2 atmospheric pressure. The β-hematin in the precipitate forms an aggregate, which is dispersed in the form of single crystal by autoclaving. Dispersing the β-hematin crystal in the form of single crystal by autoclaving is called pulverization. An average particle size of the single crystal is 20 to 1000 nm, preferably 20 to 500 nm, further preferably 50 to 300 nm and particularly preferably 50 to 200 nm.

Disclosed herein is a vaccine adjuvant composition containing an effective dose of the aforementioned β-hematin for stimulating immune response. The vaccine adjuvant refers to a substance which potentiates the effect of a vaccine when it is used in combination with the vaccine to increase the production of an antibody against an immunogen used as the vaccine in a living body. Also disclosed herein is a vaccine adjuvant composition and a vaccine composition including an allergen vaccine, which contains an allergen in an effective amount for stimulating immune response or an infection vaccine, which contains an antigen of a pathogen such as a bacterium, a virus, a rickettsia or a parasite.

The amount of β-hematin of the vaccine adjuvant composition and the vaccine composition, in the case of containing a substance (for example, aluminum hydroxide and pullulan) for connecting β-hematin and an antigen in the composition is 1 µM to 5 mM, preferably 5 µM to 3 mM, further preferably 7.5 µM to 2 mM, further preferably 10 µM to 2 mM, further preferably 10 µM to 1000 µM and further preferably 50 µM to 500 µM. Alternatively, 1 µM to 10 µM is also preferable.

When β-hematin is singly used as an adjuvant, the amount of β-hematin is 1 µM to 40 mM, preferably 50 µM to 30 mM, further preferably 100 µM to 20 mM, further preferably 500 µM to 10 mM, further preferably 1 mM to 8 mM and further preferably 3 mM to 5 mM Alternatively, 1 µM to 10 µM is also preferable.

To the vaccine adjuvant composition, a complete Freund's adjuvant, dead microorganism such as tubercle bacillus and other immuno-stimulants such as alum adjuvant may be added in addition to β-hematin.

The adjuvant can be used as an adjuvant for an allergen vaccine and for an infection vaccine.

The allergen vaccine referred to as a vaccine, by which an allergen is injected to a living body to produce an IgG antibody against an allergen to block the function of IgE or increase Type-1 helper T cells (Th1 cells) specific to an allergen in a living body, thereby reducing Type-2 helper T cells (Th2 cells) involved in an allergy symptom, and can suppress the allergy symptom by hyposensitization. The allergen vaccine is composed of allergens causing various types of allergy. Examples of the allergen to be used in combination with the vaccine adjuvant composition include, but not limited to, food allergens, house dust allergens, pollen allergens such as cedar pollen, and allergens such as body hair of animals. Specific examples of the pollen allergens include cedar pollen allergens (Cry j1, Cry j2), hogweed allergens (Amb a 1, Amb a 2, Amb a 5, Amb t 5, Amb p 5) and orchard grass allergens (Dac g 2). Specific examples of the food allergens include casein, lactalbumin, lactoglobulin, ovomucoid, ovalbumin and conalbumin. Specific examples of the house dust allergens include mite allergens (Der f 1, Der f 2, Zen 1, Der p 1, Der p 2). Of them, cedar pollen allergens such as Cry j 1 and mite allergens such as Zen 1, Der f 1 and Der f 2 are desirable.

Examples of the vaccine against an infection include an inactivated complete vaccine, a subunit vaccine and toxoid. These vaccines allow an animal to induce immunity against pathogens such as bacteria, viruses, rickettsia and parasites.

Examples of the vaccine against an infection targeting a human include influenza such as A type, A/H1N1-type and B-type influenza; and vaccines against infections such as poliovirus, Japanese encephalitis, a tubercle bacillus, human papillomavirus, malaria protozoan, SARS, avian influenza that may transmit to a human, typhoid, paratyphoid, pest, whooping cough and typhus. Furthermore, when animals except a human are targeted, examples of vaccine against infections include equine influenza, equine herpesvirus, equine encephalomeningitis virus, foot-and-mouth disease virus, rabies, feline panleucopenia, feline rhinotracheitis, infectious bovine rhinotracheitis, type-3 parainfluenza, bovine viral diarrhea, bovine adenovirus, swine parvovirus, canine adenovirus, canine distemper virus, canine parvovirus, canine parainfluenza, avian influenza, brucellosis, vibrio symptom, leptospirosis, clostridial infection and salmonellosis. Of them, vaccines against infections such as *Escherichia coli* (cow mastitis), *Staphylococcus aureus* (cow mastitis), mycoplasma (pig pneumonia), PRRS virus (pig pneumonia) and canine rabies virus are desirable.

The disclosed vaccine adjuvant composition containing β-hematin may be used alone. In this case, a vaccine adjuvant composition and the aforementioned vaccine may be separately administered to an animal. Furthermore, a vaccine adjuvant composition and a vaccine may be used as a mixture. In this case, they can be used as a vaccine composition containing β-hematin.

The animals to which the vaccine adjuvant composition and vaccine composition are to be administered are not limited and any animals having the immune system may be mentioned including mammals and birds. Examples of the mammals include humans, monkeys, cows, horses, pigs, sheep, goats, dogs, cats, marmots, rats and mice. Examples of the birds include cocks, ducks and geese. Particularly, the vaccine adjuvant composition and vaccine composition are useful as allergy vaccines and infection vaccines for humans, allergy vaccines and infection vaccines for pet animals such as dogs and cats, and infection vaccines for industrial animals such as cows, pigs and cocks.

The amount of antigen in a vaccine composition, which can be changed depending upon e.g., the type of target infection and the type of animal to be administered, is several tens to several mgs per dose.

The vaccine adjuvant composition and vaccine composition may take form such as an aseptic aqueous or non-aqueous solution, a suspension or an emulsion. Furthermore, the compositions may contain a pharmaceutically acceptable diluent such as a salt and a buffer, an auxiliary and a carrier. The vaccine composition can be injected orally, transnasally, transmucosally, intramuscularly, transdermally, subcutaneously and intradermally, and through a route such as the nasal cavity and the trachea. Furthermore, the vaccine composition may be administered by means of dropping lotion in the eyes, needling, spraying and application/rubbing, etc. Furthermore, the vaccine adjuvant composition or vaccine composition may be fed to animals by adding it to drinking water and feed. Also disclosed are drinking water and feed containing the vaccine adjuvant composition or vaccine composition.

The vaccine adjuvant composition and vaccine composition may be administered once or several times at intervals of 2 to 8 weeks.

By administering the vaccine adjuvant composition in combination with a vaccine to an animal or by administering the vaccine composition to an animal, Th1 cells increase, production of IgE, which is allergy specific antibody, reduces, production of IgG2 antibody or IgG2a antibody serving as a protective antibody in infections increase. As a result, an allergy symptom is suppressed in an animal and further the allergy symptom can be treated. Furthermore, infections can be prevented or treated.

The present invention will be more specifically explained by way of Examples; however, the present invention is not limited to these Examples.

### Example 1

This example is performed by using the following materials in accordance with the following method.

### 1. Preparation for Plasmodium protozoan (malaria protozoan; Pf) crude extract, natural hemozoin (HZ), synthetic hemozoin (sHZ) and Pf-DNA

Mycoplasma-free malaria protozoan (3D7) was kept in a medium containing 3% of type-O human erythrocytes and 10% of heat inactivated human serum, under a 5% O₂ and 5% CO₂ atmosphere (Steinman, R. M., Immunity. 29, 319-324 (2008)). After the growth stages were synchronized, a mature parasiten and a trophozoite rich in hemozoin and a parasite (about 4 to 5%) in the stage of schizont were subjected to 63% Percoll density gradient centrifugation, washed, suspended in an incomplete medium, subjected to freeze-thawing three to four times, and stored at -80°C until use.

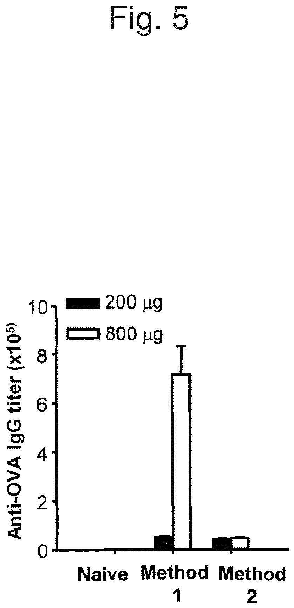

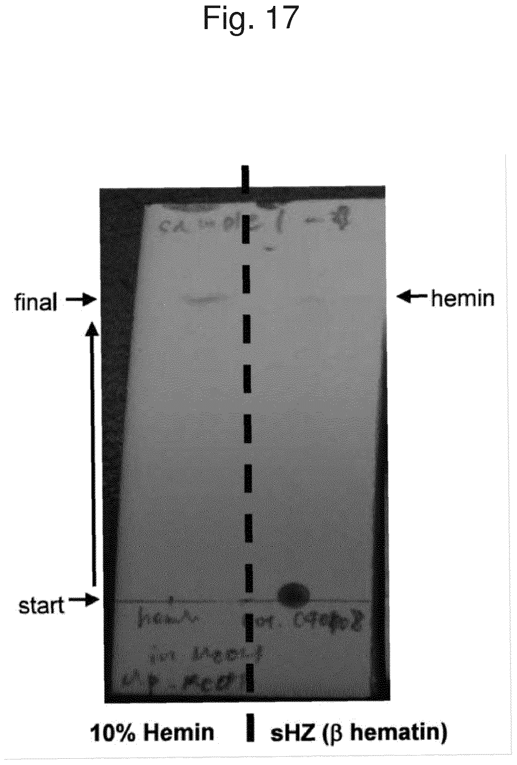

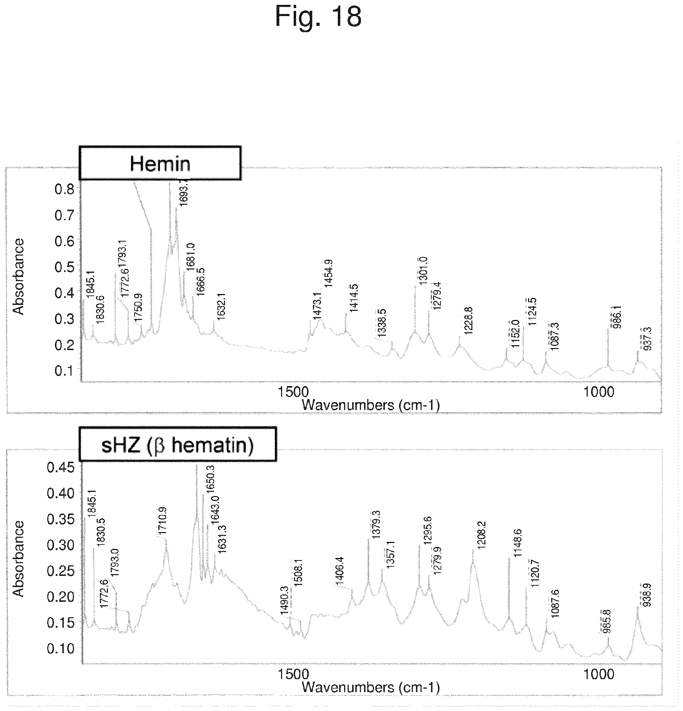

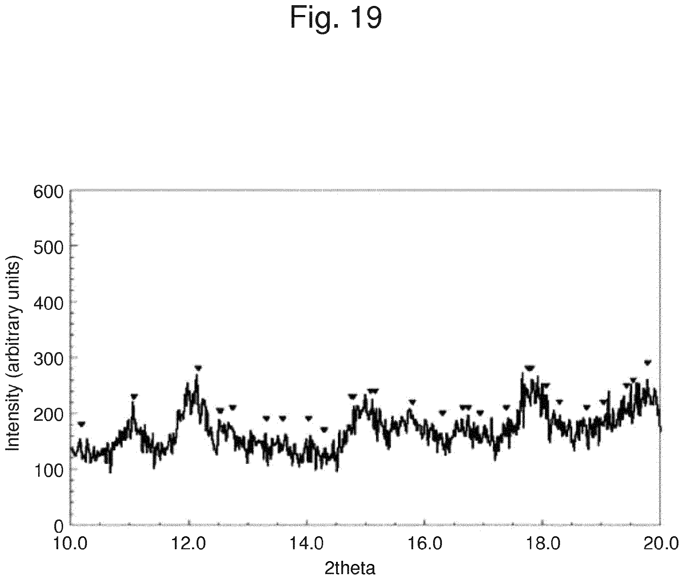

The Pf crude extraction preparation containing a large amount of hemozoin contained about 1 × 10⁹/mL of infected erythrocytes. The protein concentration was measured at 280 nm by a spectrophotometer. As the control, un-infected human erythrocytes were used. Purified hemozoin (natural HZ) was purified from Pf (3D7 strain)-infected erythrocytes (Good, M. F., Eur. J. Immunol. 39, 939-943 (2009)). Synthetic hemozoin was synthesized by two methods (Doolan, D. L., Clin. Microbiol. Rev. 22, 13-36, Table (2009)) from hemin chloride and purified. Hemin chloride was obtained from Fluka (purity measured by HPLC was 98% or more). Method 1 (acid-catalyzed method) is known to produce smaller uniform crystals (Good, M. F., Eur. J. Immunol. 39, 939-943 (2009); Coban,C. et al., Trends Microbiol. 15, 271-278 (2007)). To be brief, 45 mg of hemin chloride was dissolved in 4.5 mL of a NaOH solution and 1N hydrochloric acid (0.45 mL) was added. Thereafter, while the mixture was stirred at 60°C, acetic acid was added until pH reached 4.8. The mixture was allowed to stand still at room temperature for 16 hours to perform a reaction to form β-hematin crystals. Subsequently, centrifugation was performed to obtain a precipitate. This was washed three times with a 0.1M sodium bicarbonate buffer (pH9.1) containing 2% SDS and thereafter the buffer was completely substituted with pure water. Method 2 (anhydrous method in methanol) is known to produce larger crystals (Joffre, O. et al., Immunol. Rev. 227, 234-247 (2009)). To be brief, 52.2 mg of hemin chloride was dissolved in 2 mL of 2,6-lutidine, diluted with 10 mL of a mixture containing dimethylsulfoxide and methanol in a ratio of 1:1, shielded airtight with parafilm, etc., allowed to stand still while blocking light at room temperature for two weeks or more. Thereafter, the mixture was centrifuged and washed with 10 mL of a 0.1M sodium bicarbonate solution. A final product was washed three times with water and methanol, dried and dispersed again with pure water. A stock suspension solution in pure water was prepared for *in vivo* studies and stored at 4°C. The hemozoin concentration was obtained by weighing the dry weight of synthetic hemozoin and calculating in terms of mM or mg/mL. Most studies except those providing the results shown in Figure 4B and Figure 5 were conducted by using synthetic hemozoin prepared by Method 1. All solutions to be used in synthesis, washing and dilution were prepared with endotoxin-free pure water and stored at 4°C while blocking light. The endotoxin level was less than 0.001EU per hemozoin (nmol). Hemozoin being synthesized was confirmed by a Fourier transform type infrared spectroscopy (FT-IR). TLC was performed to confirm that the purified sample is not contaminated with residual raw material, hemin (Figures 17 and 18). Furthermore, synthetic hemozoin powder was subjected to X-ray analysis (Figure 19). Pf genomic DNA was isolated by SDS/proteinase K method and extracted by phenol-chloroform.

### 2. Mouse and immunization

### (1) Mouse

TLR9 gene-deleted C57BL/6 background mice were prepared and put in use (Good, M. F., Eur. J. Immunol. 39, 939-943 (2009); Girard, M. P. et al., RVaccine 25, 1567-1580 (2007)). The mice were obtained from CLEA Japan.

Wild-type mice and Tlr9^{-/-} mice were intraperitoneally injected with a Pf crude extract (100 µL, corresponding to about ×10⁸ protozoans) or a Pf crude extract treated with DNaseI to immunize them. Three weeks later, an antibody against the Pf crude extract in the mouse sera was measured by ELISA.

OVA (egg albumin) or HSA (human serum albumin) was used as an antigen and immunization was performed in accordance with the following immunization schedules.

### (2) Subcutaneous immunization

At Day 0, mice were subcutaneously injected with 50 µg of OVA (or 10 µg of HSA) in 200 µL of a synthetic hemozoin solution different in concentration and boosted 10 day later with 25 µg of OVA (or 10 µg of HSA) in 200 µL of the same synthetic hemozoin solution. At Day 17, a blood sample was collected to analyze OVA or HSA specific antibody response.

### (3) Intranasal immunization

Mice were anesthetized and 5 µg of OVA was administered in combination with 80 µg of synthetic hemozoin twice at a two week interval through the nasal cavity (15 µL). After 10 days later of the booster immunization, the serum, bronchovesicular washing liquid and nasal cavity secretory fluid sample were taken from the mice, and IgA was measured.

### 3. Dog and allergy model

To study the usefulness of synthetic hemozoin as an adjuvant, an allergy disease dog model was prepared.

Der f 2 (100 to 500 µg), which is one of major allergens of house dust mite (*Dermatophagoides farinae*) and alum adjuvant (50 mg) were subcutaneously administered to sensitize a beagle dog. In the beagle dog, an allergy reaction against Der f 2 can be evaluated based on a subcutaneous reaction. The reaction has a good correlation with specific IgE response against the allergen (blood level increases).

The present inventors used a 5-month old beagle dog to prepare the dog model. To this, Der f 2 (100 µg), Der f 2 (100 µg) + alum adjuvant (500 µg) or Der f 2 (100 µg) + alum adjuvant (500 µg) + synthetic hemozoin (7.5 µM) was administered and boosted 2 weeks later.

### 4. Measurement of antigen-specific T cell response

A T cell-mediated cell response was monitored by measuring secretion of a Pf crude extract-specific cytokine in the spleen cells. The spleen was taken 3 weeks after initial immunization. The spleen cells (1 × 10⁶) were seeded in a 96 well plate and stimulated with a Pf crude extract. After 72 hours, the supernatant of the cell culture was collected and the IFNγ and IL13 levels were measured by ELISA (DuoSet ELISA kit, R&D System).

### 5. Antibody ELISA

The 96-well plate was coated with 1 µg/mL OVA (egg albumin) antigen, 10 µg/mL HSA or 1 µg/mL Derf2, and ELISA was performed.

Furthermore, the plate was separately coated with a 3 µg/mL Pf-SE36 antigen, and ELISA was performed. As a secondary antibody, horseradish peroxidase (HRP) conjugate IgG, IgG1, IgG2a, IgG2b and IgG3 were used.

### 6. FESEM and particles size analysis

A slide glass was coated with poly-L-lysine and allowed to adsorb synthetic hemozoin and an image was obtained by use of an ultra-high resolution FESEM (S-4800, manufactured by Hitachi, Ltd.). The size particle distribution in the synthetic hemozoin solution was measured by a wet laser diffraction light scattering particle size distribution measuring apparatus (LA-950V2, manufactured by Horiba, Ltd.).

### 7. Statistical treatment was performed by the Student's t-test.

### Results

### 1. Pf crude extract contains a non-DNA TLR9 ligand as an adjuvant specific to a malaria antigen simultaneously administered

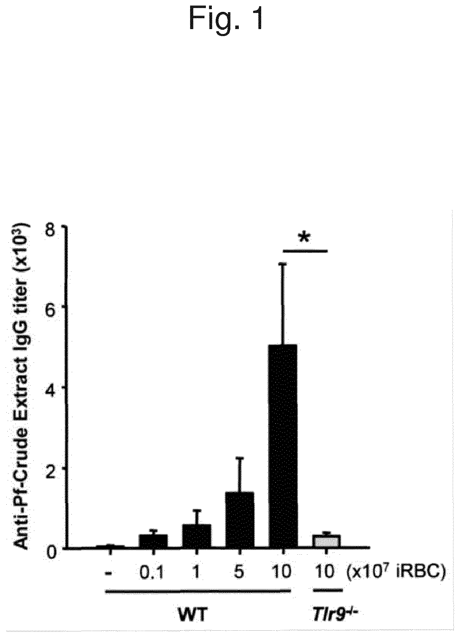

Pf infected erythrocytes were frozen and thawed to prepare a large amount of complete parasite antigen. The obtained extract was designated as a Pf crude extract. The Pf crude extract contained a product derived from both parasite and host erythrocytes. The Pf crude extract was intraperitoneally injected to mice to immunize them. After the mice was boosted with the complete parasite antigen containing no adjuvant, a serum Pf crude extract specific IgG was detected in a dose despondent manner. This was a high titer compared to that of untreated (naive) mice (Figure 1).

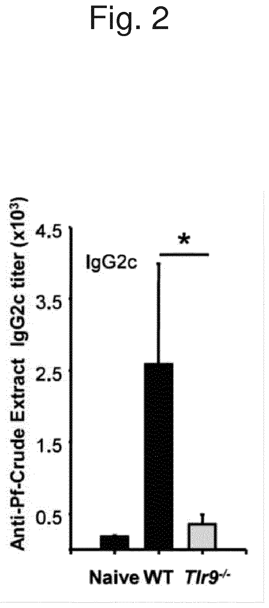

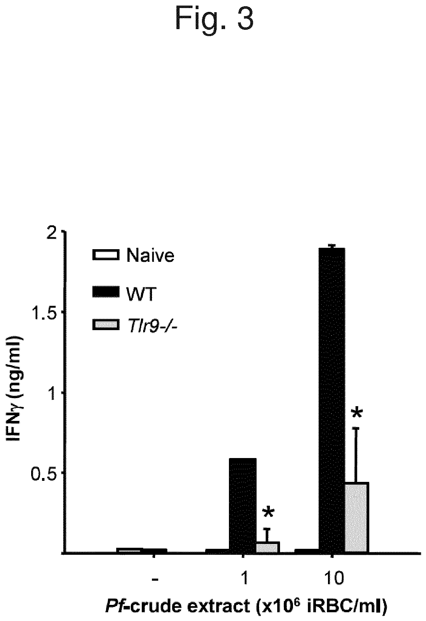

Subsequently, whether immunogenicity of the complete parasite vaccine changes in the absence of TLR9 was checked. This is because TLR9 was known to mediate activation of the natural immune system with a heat labile fraction, i.e., Pf-derived hemozoin and DNA. Accordingly, TLR9-defective mice showed significantly low serum IgG response and T cell specific IFNγ level compared to wild-type mice (Figure 1 to Figure 3). The TLR9 dependent adjuvant effect of the complete parasite antigen was not affected by DNase-I treatment and specific to a Pf antigen such as Pf-SERA5 protein. The TLR9 dependent IgG response was observed in IgG2a, IgG2 and IgG3.

### 2. Hemozoin serves as a potent adjuvant and the adjuvant activity varies depending upon the size

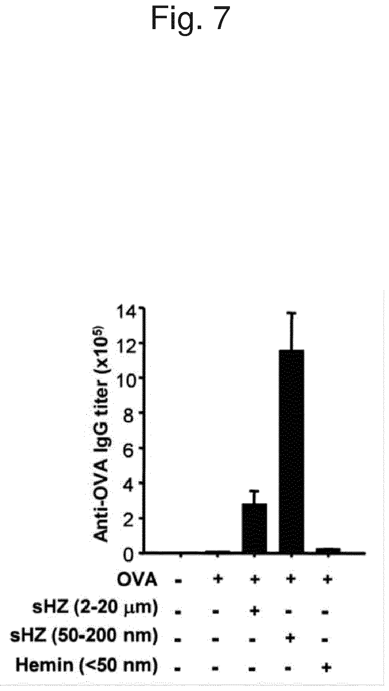

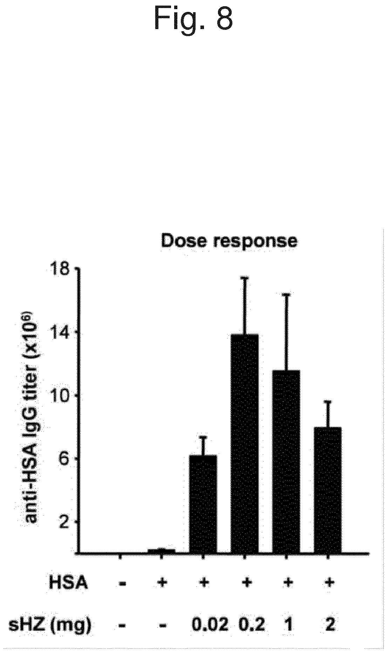

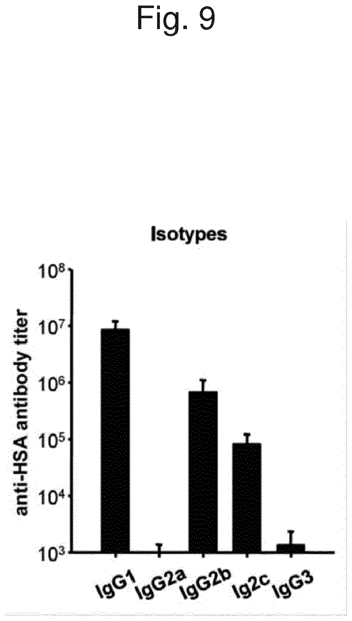

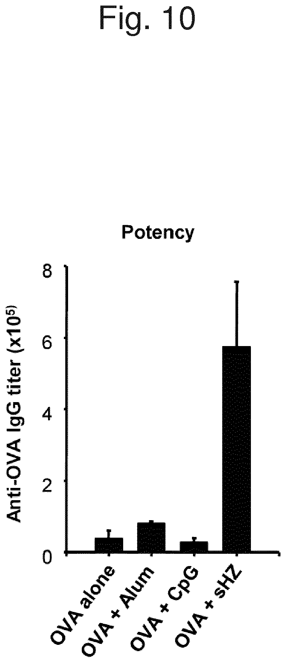

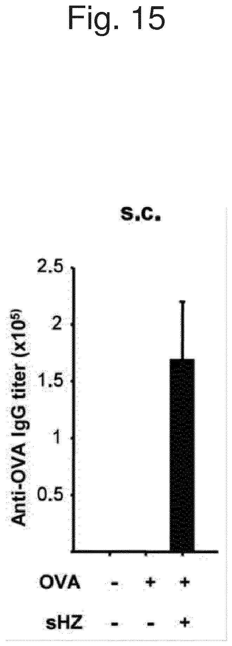

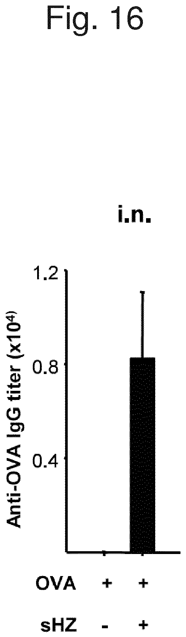

Various methods for synthesizing hemozoin were known. At first, synthesis was made by several methods and the adjuvant properties of the synthesized hemozoin samples were checked. Crystals different in adjuvant effect and having distinguishable appearances were obtained by two commonly-used methods for synthesizing hemozoin from hemin. In Method 1 where hemozoin was obtained by polymerization in the presence of acetic acid, most of the crystals obtained had a length of 50 nm to 1 µm. Whereas, in Method 2 where an organic base group was used, crystals (observed by FESEM) having a length within the range of 1 to 20 µm were obtained (Figures 4A and B). Mice were immunized by subcutaneous injection (50 µg/time) of model protein antigen OVA (egg albumin) in the presence or absence of synthetic hemozoin prepared in Method 1 or Method 2 and boosted 10 days later. As a result, the OVA specific total IgG response against hemozoin was higher in the presence of hemozoin prepared in Method 1 than in Method 2 (Figure 5). Furthermore, hemozoin prepared in Method 1 was separated in two size distributions, measured by a wet laser diffraction light scattering particle size distribution measuring apparatus (Figure 6) and used for immunization together with OVA. When administered simultaneously with OVA, synthetic hemozoin of 50 to 200 nm in size exerted a more optimum adjuvant effect than synthetic hemozoin having a larger size (2 to 20 µm) and dissolved hemin having a smaller size (50 nm or less) (Figure 7). Furthermore, the adjuvant effect of synthetic hemozoin was checked by administering through different administration routes, i.e., subcutaneous and intranasal administration. As a result, in either route, an antigen specific antibody response increased (Figures 15, 16). Furthermore, the same study was repeated with respect to HSA (10 µg/time) and the same results were obtained (Figures 8, 9, 23, 26, 27). Furthermore, dose-response was analyzed. In addition, the IgG isotype increased by synthetic hemozoin was confirmed to be mainly IgG1 in mice, followed by IgG2b and IgG2c (Figure 9). The response is strong compared to alum and a CpG DNA adjuvant (Figure 10). Accordingly, it was found that synthetic hemozoin having an optimum size of 50 to 200 nm can serve as a potent adjuvant for a protein vaccine. This coincided with other particle adjuvants, which were taken by antigen-presenting cells through receptor-mediated endocytosis.

### 3. Whether or not synthetic hemozoin can be a potent adjuvant for an allergen similarly to a malaria antigen

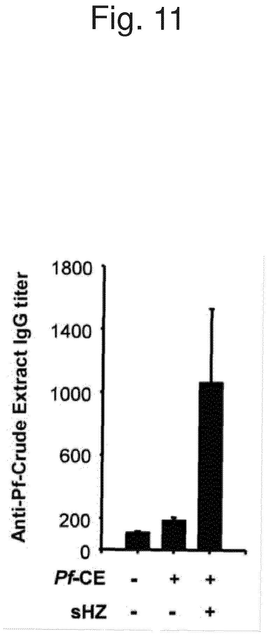

In order to check whether or not synthetic hemozoin has a potent adjuvant effect in immunization with parasite-derived Pf crude extract, mice were immunized with 10 µg of a Pf crude extract in combination with synthetic hemozoin and booster immunization was performed twice and then an anti-Pf crude extract specific antibody response was measured. The IgG level (mainly, IgG2c and IgG1, although not shown in Figure) against the Pf crude extract was increased by synthetic hemozoin (Figure 11).

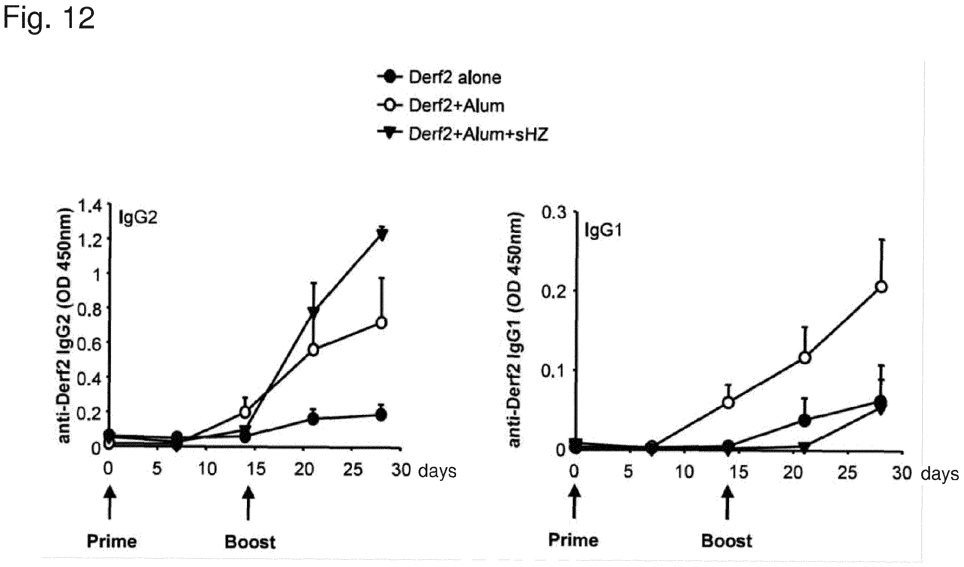

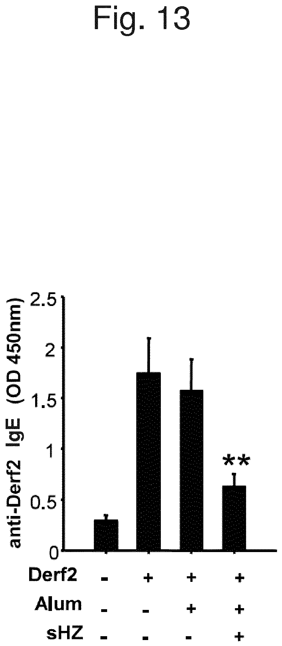

Whether or not synthetic hemozoin can be used in animals other than mice was evaluated by use of allergy dog models. The present inventors injected the beagle dog models with Der f 2, Der f 2 + alum adjuvant or Der f 2 + alum adjuvant + synthetic hemozoin, and boosted 2 weeks later. The Der f 2 specific IgG levels in the sera of these dogs were measured by ELISA over time. In the group administered in combination with synthetic hemozoin, IgG2 antibody titer significantly increased after booster immunization; however, a significant increase of IgG1 was not observed. The immune response was analogous to a Th1 response (Figure 12). Furthermore, the dogs after immunization were sensitized again with Der f 2. In the group in which synthetic hemozoin was administered in combination, an increase of a Der f 2 specific IgE antibody titer is significantly low compared to the group in which synthetic hemozoin is not administered in combination (Figure 13). From these data, it was suggested that in the allergy disease dog models, synthetic hemozoin may possibly function as a potent Th1-like adjuvant.

These data indicate that synthetic hemozoin present together with a substance such as aluminum hydroxide, which connects it to an antigen, is a potent Th1 adjuvant in dog models. Whereas, in mouse models, when it is used singly, it was a Th2-dominant adjuvant. In an experiment using non-human primates, synthetic hemozoin was demonstrated to be a potential Th1 type adjuvant when a protein vaccine is administered, similar to the case of CpG DNA.

Natural hemozoin purified from *P. falciparum* culture is reported to be identical with synthetic hemozoin (Pagola, S. et al, Nature 404, 307-310.). If purification and synthesis methods differ, the resulting crystals and crystal aggregates vary in size in the range of 20 nm to 20 µm. They show different adjuvant activities depending upon the property of unit crystals (primary particles) and presence or absence of aggregates (secondary particles) as shown in Figure 5.

Furthermore, in the Example, it was shown that synthetic hemozoin exhibits potent adjuvant activity if it is synthesized by a specifically optimized method and it has an optimum size. The adjuvant activity of synthetic hemozoin for a protein vaccine was confirmed in different antigen models OVA and HSA through different administration routes such as subcutaneous injection, intraperitoneal injection and intranasal administration. Different from CpG DNA exhibiting species specificity, hemozoin was confirmed in several types of vaccine animal models including mice, dogs and non-human primates (Figures 5, 7 to 13, 15 and 16). In mice, when immunization was performed by use of synthetic hemozoin alone as an adjuvant, IgG1 is dominantly induced as an adjuvant effect. The results demonstrate that hemozoin induces Th2 type immune response in mice (Figures 5, 7 to 11, 15 and 16). Whereas, when synthetic hemozoin and a substance (for example, aluminum hydroxide and pullulan), which connects it to an antigen were used as an adjuvant in dogs, an IgG2 response is dominantly induced. This demonstrates that Th1 type immune response was induced. Use of synthetic hemozoin to dog allergy models extremely strongly reduces an allergen specific IgE response (Figures 12 and 13). The results demonstrate that synthetic hemozoin can be effectively used as an adjuvant against allergy. Also in non-human primates, synthetic hemozoin induced a malaria antigen-specific IgG response. It was found that synthetic hemozoin can be used as a novel vaccine adjuvant for complete whole blood level vaccine.

In this Example, it was shown that hemozoin controls the TLR9 pathway to exhibit inflammation and adjuvant activity, thereby serving as a therapy target for treating malaria infection, and thus it is useful for developing a vaccine adjuvant.

### Example 2

### (1) Mouse

Six to twelve week-old wild-type mice C57BL/6 and Balb/c background available from CLEA Japan were used.

### (2) Antigen and adjuvant

Immunization was performed by use of OVA (egg albumin) or HSA (human serum albumin) as an antigen in accordance with the following immunization schedules.

Synthetic hemozoin was synthesized from hemin chloride used as a raw material according to the method described in Example 1. At this time, hemin chloride from Fluka and hemin chloride from TCI (Tokyo Chemical Industry Co., Ltd.) were used. Furthermore, two types of synthetic hemozoin samples obtained from different synthesis lots were used to check difference between the lots.

Synthetic hemozoin particles were fractionated based on the particle diameter. Large synthetic hemozoin particles (2 to 50 µm) and small synthetic hemozoin particles (20 to 500 nm) were used.

### (2) Subcutaneous immunization

At Day 0, mice were subcutaneously injected with 50 µg of OVA (or 10 µg of HSA) in 200 µL of a synthetic hemozoin solution (different in concentration) diluted with PBS (manufactured by Sigma) and boosted 10 day later with 25 µg of OVA (or 10 µg of HSA) in 200 µL of synthetic hemozoin solution diluted with PBS having the same concentration as above. At Day 17, blood samples were collected and OVA or HSA specific antibody response in the sera were analyzed by ELISA method.

### (3) Intraperitoneal immunity

At Day 0, mice were intraperitoneally injected with 50 µg of OVA (or 10 µg of HSA) in 200 µL of a synthetic hemozoin solution (different in concentration) diluted with PBS (manufactured by Sigma) and boosted 10 day later with 25 µg of OVA (or 10 µg of HSA) in 200 µL of synthetic hemozoin solution diluted with PBS having the same concentration as above. At Day 17, blood samples were collected and OVA or HSA specific antibody response in the sera were analyzed by ELISA method.

### Results

1. The adjuvant effect of synthetic hemozoin varies depending upon the particle size (or particle-size distribution and particle properties) and reproducible even if lots differ.

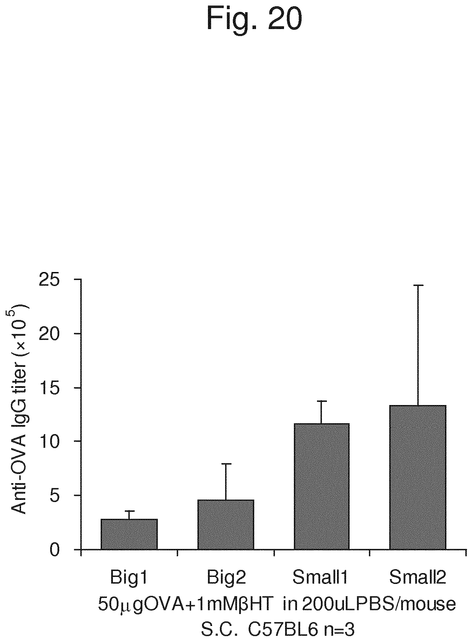

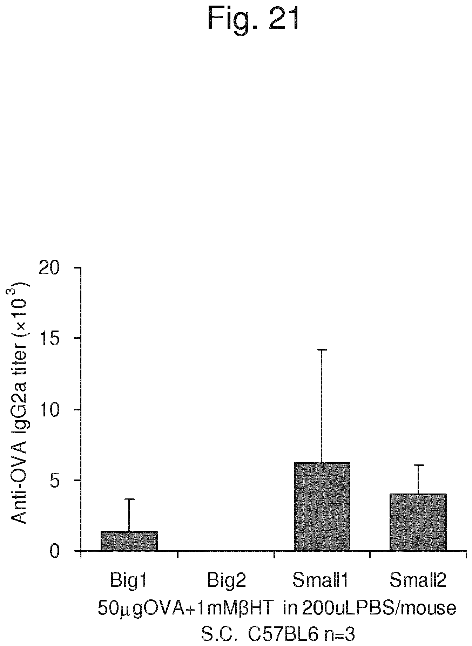

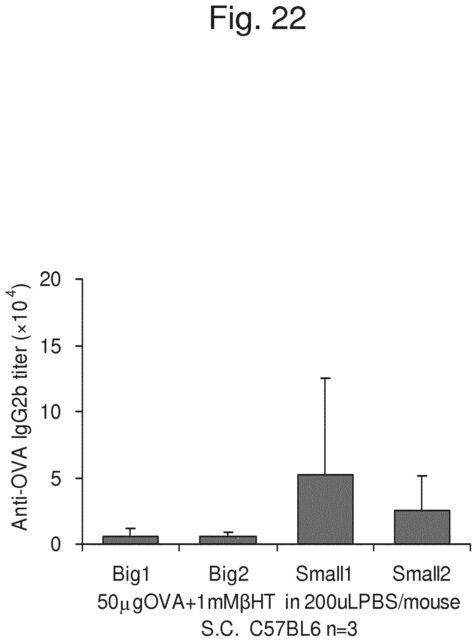

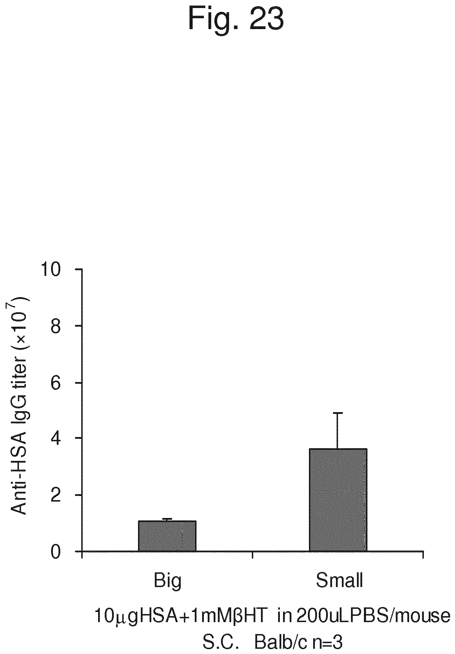

Hemozoin was synthesized in several lots by polymerization in the presence of acetic acid (Method 1). The difference in adjuvant effect was compared between the lots and between the average particle sizes (large or small, i.e., presence or absence of aggregation). As a result, a fraction of β-hematin crystal having a small average particle size had a higher IgG producibility in an antigen specific antibody response in a plurality of lots (Figures 20 to 27). The same results were obtained when HSA antigen other than OVA was used (Figure 23). Furthermore, the same results were obtained in the case of a sub type (Figures 20 to 22).
2. Adjuvant effect of synthetic hemozoin varies depending upon the particle diameter (or particle properties) similarly in the case of intraperitoneal administration but does not vary depending upon the maker of raw material.

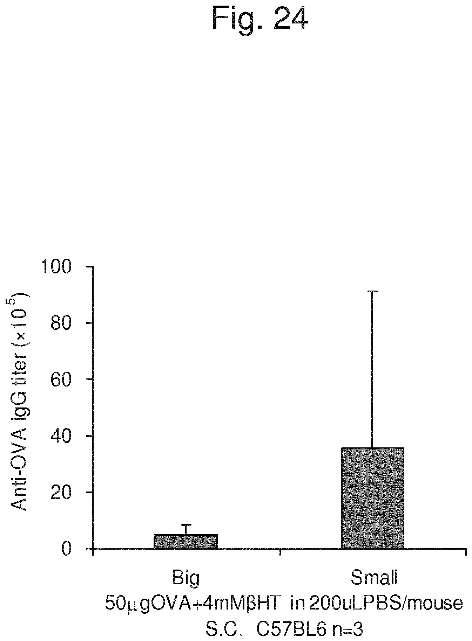

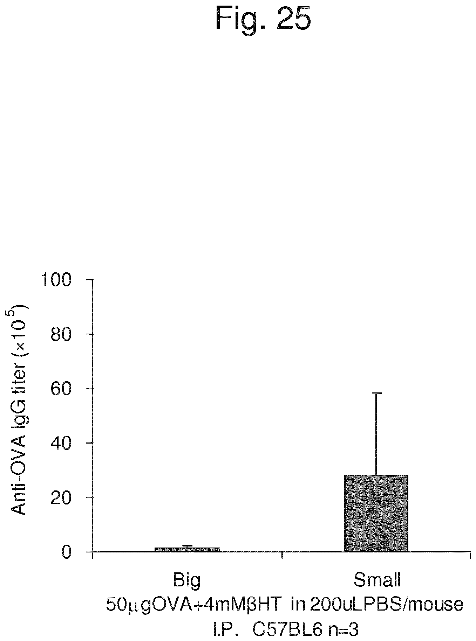

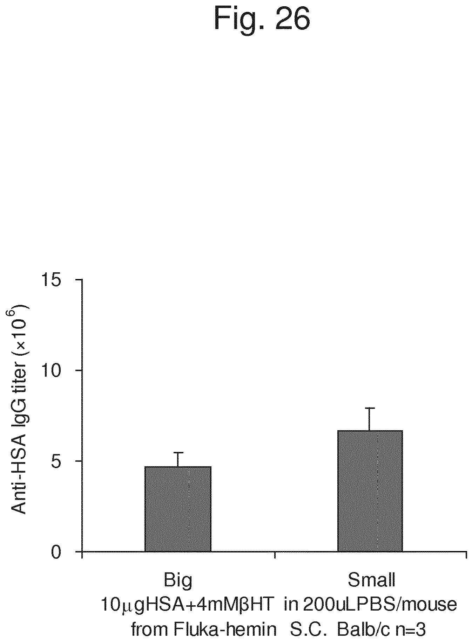

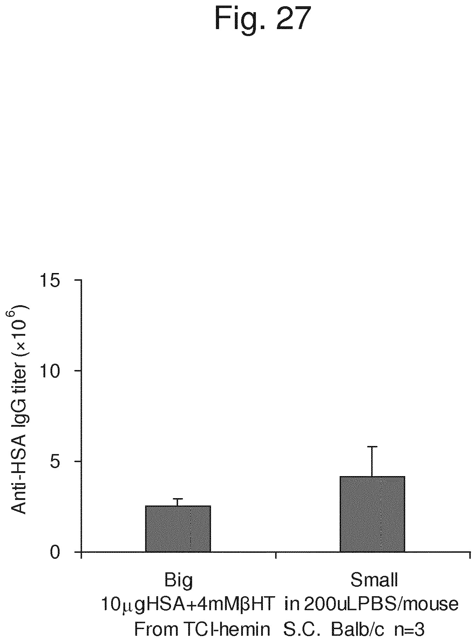

Similarly to subcutaneous administration, also in the intraperitoneal administration, the smaller the average particle size of the fraction, the higher the antigen specific IgG antibody producibility (Figures 24 and 25). Furthermore, β-hematin samples synthesized from hemin materials manufactured by two makers, Fluka and TCI, both exerted high adjuvant effects, and fractions thereof having a smaller particle size both exerted high adjuvant effects (Figures 26 and 27).

### Example 3 Effect of β-hematin autoclaved

Hemin chloride (45 mg) (Fuluka) was dissolved in a 4.5 mL NaOH solution and 1N hydrochloric acid (0.45 mL) was added. Thereafter, to this, acetic acid was added at 60°C while stirring until pH reached 4.8. The mixture was kept still at room temperature overnight for reaction to form a β-hematin crystal. Subsequently, centrifugation was performed to obtain a precipitate. This was washed three times with a 2% SDS containing 0.1M sodium bicarbonate buffer (pH9.1) and then the buffer was completely substituted with pure water. The precipitate was autoclaved 121°C for 20 minutes. As the autoclave, HICLAVE HVP-50 manufactured by Hirayama Manufacturing Corporation was used.

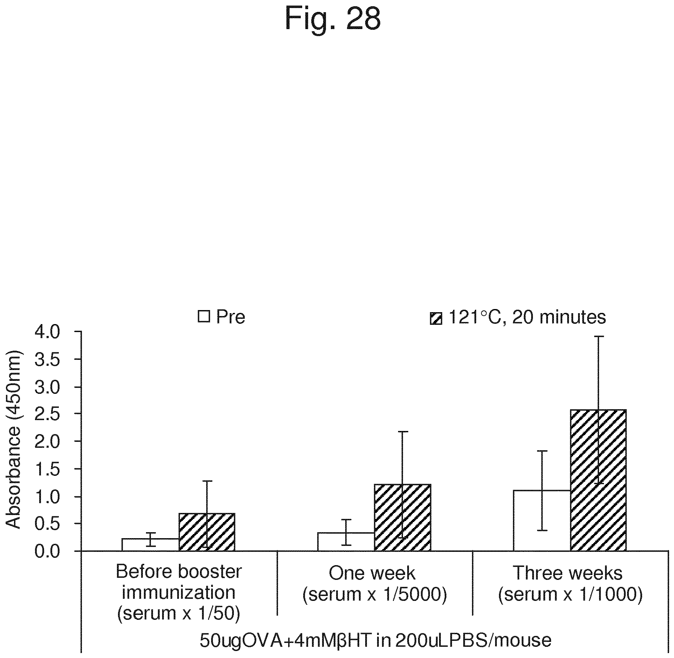

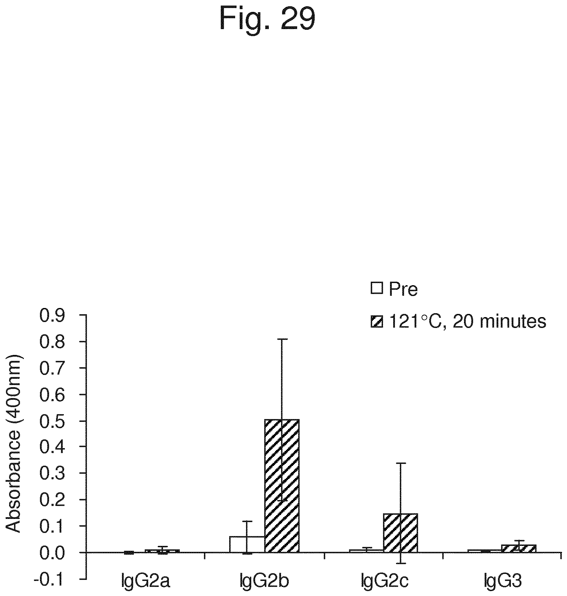

Mice were subcutaneously immunized with the precipitate mentioned above (not autoclaved) and a sample obtained by autoclaving the precipitate, in accordance with the "subcutaneous immunization method" set forth in paragraph 2. (2) of Example 1, and the total IgG in blood of each of the mice was measured in accordance with the "antibody ELISA method" set forth in paragraph 5 of Example 1. The measurement results of the total IgG level in the sera taken before the booster immunization, one or four weeks after the booster immunization are shown in Figure 28. In Figure 28, the sample not autoclaved is indicated by "pre" (the same is applied to Figure 29). As shown in Figure 28, in the sample obtained by autoclaving the precipitate at 121°C for 20 minutes, a higher blood total IgG level was observed.

Furthermore, similarly IgG2a, IgG2b, IgG2c and IgG3 levels in mouse blood were measured. The measurement results of IgG2a, IgG2b, IgG2c and IgG3 levels in the serum taken three weeks after booster immunization are shown in Figure 29. As is shown in Figure 29, each of blood levels of IgG2a, IgG2b, IgG2c and IgG3 increased in the sample obtained by autoclaving the precipitate at 121°C for 20 minutes. Particularly, the blood level in IgG2a was remarkable.

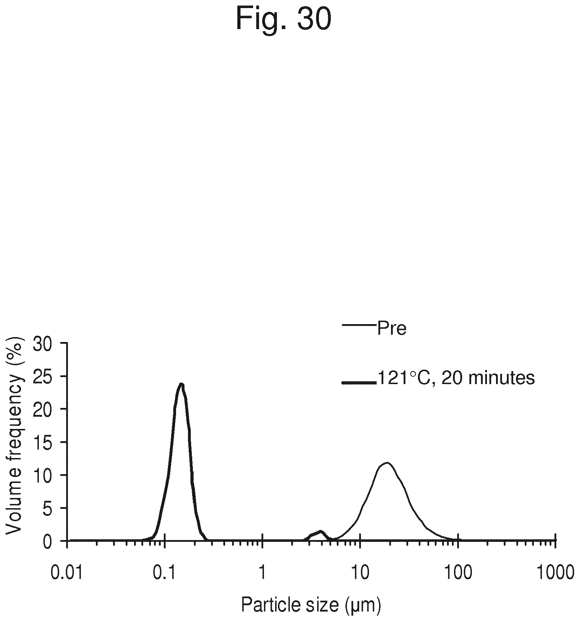

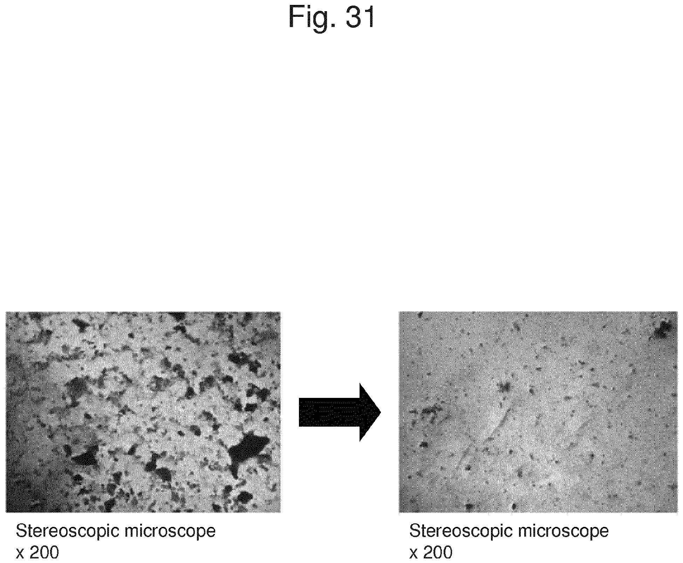

Furthermore, particle size distributions of the samples were measured and the shapes of particles were observed by a stereoscopic microscope in accordance with the "FESEM and particle-size analysis method" set forth in paragraph 6 of Example 1. Figure 30 shows the particle-size distribution of the obtained samples and Figure 31 shows microscopic images observed. In a sample obtained by autoclaving the precipitate at 121°C, for 20 minutes, the size of the particles was reduced.

### Industrial Applicability

The vaccine adjuvant composition and vaccine composition can be used for prevention and treatment of allergy diseases and infections of animals including humans in the fields of medicine and veterinary medicine.

## Claims

1. A method for preparing a vaccine adjuvant composition comprising a β-hematin crystal, comprising dissolving hemin chloride in an aqueous NaOH solution, adding a small amount of hydrochloric acid to the resultant solution, adding dropwise acetic acid to the resultant solution at room temperature to 60°C to adjust the pH to 4 to 6, keeping the resultant solution mixture still at room temperature to 40°C for 1 to 24 hours for reaction to occur, centrifuging the resultant mixture to obtain a β-hematin crystal, washing the β-hematin crystal with an SDS-comprising weakly basic solution of about pH9, and pulverizing the washed β-hematin crystal by autoclaving to obtain a β-hematin crystal having an average particle size of 50 to 500 nm.

2. The method for preparing a vaccine adjuvant composition comprising a β-hematin crystal according to claim 1, wherein the washed β-hematin crystal is pulverized by autoclaving to obtain a β-hematin crystal having an average particle size of 50 to 300 nm.

3. The method for preparing a vaccine adjuvant composition comprising a β-hematin crystal according to claim 1, wherein the washed β-hematin crystal is pulverized by autoclaving to obtain a β-hematin crystal having an average particle size of 50 to 200 nm.

## Patentansprüche

1. Verfahren zur Herstellung einer Vakzinen-Adjuvans-Zusammensetzung, die einen β-Hämatin-Kristall umfasst, umfassend das Auflösen des Häminchlorids in einer wässrigen NaOH-Lösung, Zusetzen einer kleinen Menge Salzsäure zu der resultierenden Lösung, Zutropfen von Essigsäure zu der resultierenden Lösung bei Raumtemperatur bis 60 °C, um den pH auf 4 bis 6 einzustellen, weiteres Halten des resultierenden Lösungsgemischs bei Raumtemperatur bis 40 °C über 1 bis 24 h hinweg, damit die Reaktion erfolgt, Zentrifugieren des resultierenden Gemischs, um einen β-Hämatin-Kristall zu erhalten, Waschen des β-Hämatin-Kristalls mit einer SDS-haltigen, schwach basischen Lösung mit etwa pH 9 und Pulverisieren des gewaschenen β-Hämatin-Kristalls durch Autoklavieren, um einen β-Hämatin-Kristall mit einer mittleren Partikelgröße von 50 bis 500 nm zu erhalten.

2. Verfahren zur Herstellung einer Vakzinen-Adjuvans-Zusammensetzung, die einen β-Hämatin-Kristall umfasst, nach Anspruch 1, wobei der gewaschene β-Hämatin-Kristall durch Autoklavieren pulverisiert wird, um einen β-Hämatin-Kristall mit einer mittleren Partikelgröße von 50 bis 300 nm zu erhalten.

3. Verfahren zur Herstellung einer Vakzinen-Adjuvans-Zusammensetzung, die einen β-Hämatin-Kristall umfasst, nach Anspruch Anspruch 1, wobei der gewaschene β-Hämatin-Kristall durch Autoklavieren pulverisiert wird, um einen β-Hämatin-Kristall mit einer mittleren Partikelgröße von 50 bis 200 nm zu erhalten.

## Revendications

1. Procédé pour préparer une composition d'adjuvant de vaccin comprenant un cristal de β-hématine, comprenant la dissolution de chlorure d'hémine dans une solution aqueuse de NaOH, l'addition d'une petite quantité d'acide chlorhydrique à la solution résultante, l'addition goutte à goutte d'acide acétique à la solution résultante entre la température ambiante et 60°C pour ajuster le pH entre 4 et 6, le maintien du mélange en solution ainsi obtenu au repos entre la température ambiante et 40°C pendant 1 à 24 heures pour qu'il se produise une réaction, la centrifugation du mélange résultant pour que soit obtenu un cristal de β-hématine, le lavage du cristal de β-hématine avec une solution faiblement basique contenant du SDS à un pH d'environ 9, et la pulvérisation du cristal de β-hématine lavé par traitement en autoclave pour que soit obtenu un cristal de β-hématine ayant une granulométrie moyenne de 50 à 500 nm.

2. Procédé pour préparer une composition d'adjuvant de vaccin comprenant un cristal de β-hématine selon la revendication 1, dans lequel le cristal de β-hématine lavé est pulvérisé par traitement en autoclave pour que soit obtenu un cristal de β-hématine ayant une granulométrie moyenne de 50 à 300 nm.

3. Procédé pour préparer une composition d'adjuvant de vaccin comprenant un cristal de β-hématine selon la revendication 1, dans lequel le cristal de β-hématine lavé est pulvérisé par traitement en autoclave pour que soit obtenu un cristal de β-hématine ayant une granulométrie moyenne de 50 à 200 nm.
